# EUROPÄISCHE PATENTSCHRIFT

(11) **EP 0 832 867 B1**
(45) Veröffentlichungstag und Bekanntmachung des Hinweises auf die Patenterteilung: **05.01.2000**
(21) Anmeldenummer: 97116716.8
(22) Anmeldetag: 25.09.1997
(51) Int. Cl.: C07C 11/06, C07C 11/08, C07C 6/04

(54) **Verfahren zur Herstellung von Propen**
Process for the preparation of propene
Procédé pour la préparation du propène

(30) Priorität: 27.09.1996 DE 19640026; 17.09.1997 DE 19740895
(43) Veröffentlichungstag der Anmeldung: 01.04.1998
(73) Patentinhaber: BASF AKTIENGESELLSCHAFT, 67056 Ludwigshafen (DE)
(72) Erfinder: Schwab, Peter, Dr., 67098 Bad Dürkheim (DE); Höhn, Arthur, Dr., 67281 Kirchheim (DE)
(74) Vertreter: Isenbruck, Günter, Dr.

(56) Entgegenhaltungen:
- EP-A- 0 691 318

## Beschreibung

Die Erfindung betrifft ein Verfahren zur Herstellung von Propen durch Metathese von Olefinen.

Die Olefinmetathese (Disproportionierung) beschreibt in ihrer einfachsten Form die reversible, metallkatalysierte Umalkylidenierung von Olefinen durch Bruch und Neuformierung von C=C-Doppelbindungen. Dabei werden beispielsweise Olefine der Formeln R¹-CH=CH-R² und R³-CH=CH-R⁴ reversibel zu Olefinen der Formeln R¹-CH=CH-R³ und R²-CH=CH-R⁴ umgesetzt. Bei der Metathese acyclischer Olefine unterscheidet man zwischen Selbstmetathese, bei der ein Olefin in ein Gemisch zweier Olefine unterschiedlicher molarer Massen übergeht, und Kreuz- oder Co-Metathese, bei der zwei unterschiedliche Olefine reagieren. Ein Beispiel für eine Selbstmetathese ist die Umsetzung von zwei Molekülen Propen zu Ethen und 2-Buten, wie sie beispielsweise nach dem Phillips-Triolefin-Verfahren ausgeführt wird, siehe Hydrocarbon Processing, Band 46, Nr. 11, November 1967, S. 232. Ein Beispiel für eine Kreuz-Metathese ist die Umsetzung von Propen und 1-Buten zu Ethen und 2-Penten. Ist einer der Reaktionspartner Ethen, so spricht man üblicherweise von einer Ethenolyse.

Die Metathesereaktionen erfolgen in Gegenwart von Katalysatoren. Hierzu eignen sich prinzipiell homogene und heterogene Übergangsmetallverbindungen, insbesondere solche der VI bis VIII Nebengruppe des Periodensystems der Elemente, wie auch homogene und heterogene Katalysatorsysteme, in denen diese Verbindungen enthalten sind.

Aus DE-A-19 40 433 ist die Metathese von 1-Buten mit 2-Buten zu Propen und 2-Penten bekannt, wobei Re₂O₇/Al₂O₃ als Katalysator verwendet wird. Das gebildete 2-Penten wird mit Natriumhydrid auf Kaliumcarbonat und Ethen zu Heptenen weiter umgesetzt.

Die Methathese von 1-Buten und 2-Buten zu Propen und 2-Penten wird in K.L. Anderson, T.D. Brown, Hydrocarbon Processing, Band 55, Nr.8, August 1976, S.119-122 als Nebenreaktion bei der Synthese von Isoamylen aufgeführt.

Aus EP-A-0 304 515 ist ein Metatheseverfahren zur Umsetzung von 1-Buten mit 2-Buten zu Propen und Pentenen bekannt, das in einer Reaktivdestillationsvorrichtung mit Re₂O₇/Al₂O₃ als Katalysator ausgeführt wird.

Aus US 3,526,676 ist die Metathese von 1-Buten mit 2-Buten zu Propen und Penten bekannt. Sie wird an MoO₃ und CoO auf Al₂O₃ durchgeführt.

Aus US 3,785,957 ist ein Verfahren zur Herstellung von Treibstoff mit hoher Octanzahl bekannt. Dabei wird ein olefinischer Treibstoff mit Ethylen disproportioniert und der Austrag aufgetrennt in einen Propylenstrom, einen Butenstrom, einen C₅- oder C₅₋₆-Olefinstrom und einen C₆₊- oder C₇₊- Treibstoffstrom. Der C₅- oder C₅₋₆-Olefinstrom wird mit Ethen an einem WO₃/SiO₂-Festbettkatalysator disproportioniert, um Propylen und Butene zu erhalten. Das erhaltene Propylen wird disproportioniert zu Ethylen und Butenen und die Butene werden mit Isobutan alkyliert.

Aus US 3,767,565 ist ein Verfahren zur Erhöhung der Octanzahl von Treibstoff bekannt, wobei ein C₅-Schnitt eines olefinischen Treibstoff mit Ethylen in Gegenwart eines Katalysators aus WO₃/SiO₂ und MgO umgesetzt wird zu Ethylen, Propylen, n-Butenen und Isobutenen. Das erhaltene Propylen wird disproportioniert und die erhaltenen n-Butene werden mit Isobutan alkyliert.

Aus EP-A1-0 691 318 ist ein Olefinmetatheseverfahren bekannt, bei dem C₅-Olefine und Ethylen in Gegenwart eines Katalysators zu gemischten C₄-Olefinen und Propen umgesetzt werden. So wird 2-Methyl-2-buten mit Ethen zu Isobuten und Propen umgesetzt. Ein Gemisch aus 2-Pentenen und 2-Methyl-2-buten wird zu einem Gemisch aus 1-Buten, Isobuten und Propen umgesetzt. Auch die Umsetzung von 2-Penten mit Ethen zu Propen und 1-Buten wird erwähnt.

Ein Verfahren zur Herstellung von Propen in hoher Ausbeute durch Umsetzung von 1-Buten und 2-Buten ist nicht bekannt.

Ein Verfahren zur Herstellung von Propen in hoher Ausbeute aus 2-Buten ohne Verwendung eines Überschusses an Ethen ist nicht bekannt.

Eine Aufgabe der Erfindung ist die Bereitstellung eines Verfahrens zur Herstellung von Propen in hoher Ausbeute aus 1-Buten und 2-Buten.

Eine weitere Aufgabe der vorliegenden Erfindung ist die Bereitstellung eines Verfahrens zur Herstellung von Propen aus 2-Buten, bei dem nicht mit einem Ethen-Überschuß gearbeitet werden muß.

Eine weitere Aufgabe der vorliegenden Erfindung ist die Bereitstellung eines Verfahrens zur Gewinnung von Propen aus 1-Buten-armen C₄-Strömen mit möglichst geringem Ethenbedarf.

Die Aufgaben werden erfindungsgemäß gelöst durch ein Verfahren zur Herstellung von Propen und 1-Buten durch
a) Umsetzung von 1-Buten und 2-Buten zu Propen und 2-Penten in Gegenwart eines Metathesekatalysators, der mindestens eine Verbindung eines Metalls VIb, VIIb oder VIII Nebengruppe des Periodensystems der Elemente enthält,
b) anschließendes Trennen des gebildeten Propens und 2-Pentens,
c) anschließende Umsetzung des 2-Pentens mit Ethen zu Propen und 1-Buten in Gegenwart eines Metathesekatalysators, der mindestens eine Verbindung eines Metalls der VIb, VIIb oder VIII Nebengruppe des Periodensystems der Elemente enthält,
d) anschließendes Trennen des gebildeten Propens und 1-Butens,
e) anschließendes Rückführen des gebildeten 1-Butens in Schritt a).

Als Nettoreaktion ergibt sich somit die Umsetzung von 2-Buten mit Ethen zu 2 Molekülen Propen. Bei der Umsetzung von 2-Penten mit Ethen werden gemäß einer Ausführungsform der Erfindung formal nur equimolare Mengen an Einsatzstoffen benötigt, um mit hoher Ausbeute zu den Produkten zu gelangen. Somit kann im Gegensatz zum umgekehrten Trioolefin-Verfahren die eingesetzte Ethenmenge geringgehalten werden.

Beide Metatheseschritte können als Reaktivdestillation ausgeführt werden, wie sie nachstehend beschrieben ist.

1-Buten und 2-Buten können in der Umsetzung als reine Stoffe eingesetzt werden gemäß einer Ausführungsform der Erfindung. Gemäß einer anderen Ausführungsform der Erfindung werden die Butene in Form eine C₄-Stroms eingesetzt, der beispielsweise aus einem Cracker, insbesondere Steamcracker, oder einer Raffination stammt. Der C₄-Strom kann dabei neben den Butenen C₄-Alkane enthalten. Gemäß einer Ausführungsform der Erfindung wird ein C₄-Strom verwendet, der aus Raffinat II besteht. Raffinat II ist dabei eine Fraktion aus 1-Buten, cis/trans-2-Buten, Isobuten sowie n-Butan und Isobutan. Beispielsweise kann Raffinat II 80 - 85 Gew.-% Olefine und 15 - 20 Gew.-% Butane enthalten, mit beispielsweise 25 - 50 Gew.-% 1-Buten, 30 bis 55 Gew.-% 2-Buten, maximal 1 - 2 Gew.-% Isobuten. Gemäß einer Ausführungsform der Erfindung besitzt der eingesetzte C₄-Strom einen Anteil an Butenen von 20 bis 100, vorzugsweise 50 bis 90, insbesondere 70 bis 90 Gew.-%. Das Verhältnis von 1-Buten zu 2-Buten beträgt dabei 10:1 bis 1:10, vorzugsweise 3:1 bis 1:3, insbesondere 2:1 bis 1:2. Gemäß einer Ausführungsform der Erfindung kann der C₄-Strom geringe Mengen anderer Kohlenwasserstoffe enthalten.

Gemäß einer Ausführungsform der Erfindung kann als Ausgangsstoff jeder Strom verwendet werden, der 1-Buten und 2-Buten enthält. Gemäß einer Ausführungsform der Erfindung kann dabei das 1-Buten aus der erfindungsgemäßen Synthese selbst stammen und mit zugeführtem 2-Buten gemischt werden.

Der eingesetzte C₄-Feedstrom wird vorzugsweise vor dem Einsatz im erfindungsgemäßen Verfahren vorgereinigt, um gegebenenfalls vorliegende Spuren an Wasser, Sauerstoff bzw. Oxygenates, Schwefel oder Schwefel enthaltenden Verbindungen, Stickstoff, Phosphor oder Halogen speziell Chloriden zu entfernen. Vorzugsweise erfolgt die Entfernung durch Leiten des C₄-Feedstroms über Absorbermaterialien, wie Zeolithe und zeolithanaloge Phosphate, hochoberflächige Oxide des Siliziums, Aluminiums, Titans, Zirkons, Bleicherden, Tone, Hydrotalcite, hochoberflächige Phosphate, Aktivkohlen und Kohlenstoffmolekularsiebe, sowie organische Polymere und Ionenaustauscherharze, bevorzugt NaX-Molsieb. Die Absorbermaterialien liegen vorzugsweise als Schutzbett (guard bed) vor.

Einsetzbare Verfahren zur Adsorption und adsorptiven Reinigung sind beispielsweise beschrieben in W. Kast, Adsorption aus der Gasphase, VCH, Weinheim (1988). Der Einsatz von zeolithischen Adsorbentien wird erläutert bei D.W. Breck, Zeolite Molecular Sieves, Wiley, New York (1974). Die Entfernung von speziell Acetaldehyd aus C₃ bis C₁₅-Kohlenwasserstoffen in flüssiger Phase ist im EP-A-0 582 901 beschrieben. Die in der vorstehenden Literatur angeführten Verfahren können vorliegend eingesetzt werden. So bringt man vorzugsweise den Eduktstrom in gasförmiger, flüssiger oder überkritischer Phase mit den Adsorbentien in Kontakt.

Neben der Umsetzung von 1-Buten und 2-Buten zu Propen und 2-Penten kann ein geringer Anteil an 3-Hexen und Ethen als Nebenprodukt erhalten werden. Zudem können auch geringe Mengen höhersiedender Verbindungen vorliegen.

Die geringen Mengen an Nebenprodukten, die gemäß einer Ausführungsform der Erfindung 1 bis 20 Gew.-%, vorzugsweise 1 bis 5 Gew.-%, bezogen auf die Mengen an gebildetem 2-Penten ausmachen, stören bei der nachfolgenden Umsetzung nicht, so daß gemäß einer Ausführungsform der Erfindung keine Reinigung des 2-Pentens von diesen Nebenprodukten vor der weiteren Umsetzung erforderlich ist. Gemäß einer Ausführungsform der Erfindung wird das 2-Penten in reiner Form in der zweiten Umsetzung eingesetzt.

Somit werden unter dem Ausdruck "2-Penten" auch solche Gemische verstanden, die neben 2-Penten geringe Mengen an Hexenen, insbesondere 3-Hexen, und anderen höhersiedenden Verbindungen enthalten.

Entsprechend wird unter dem Ausdruck "Butene", wie "1-Buten" und "2-Buten" auch ein Gemisch verstanden, das neben dem Buten oder den Butenen C₄-Alkane, insbesondere Butane enthält.

Mehrere Ausführungsformen der Erfindung werden nachstehend anhand der Zeichnung erläutert, in der
- Figur 1: eine schematische Darstellung einer Ausführungsform des erfindungsgemäßen Verfahrens zeigt,
- Figur 2: eine weitere Ausführungsform des erfindungsgemäßen Verfahrens zeigt und
- Figur 3: eine weitere Ausführungsform des erfindungsgemäßen Verfahrens zeigt.

Dabei bedeuten die in den Figuren verwendeten Abkürzungen folgendes:

| | |
|---|---|
| 1-Bu | 1-Buten |
| 2-Bu | 2-Buten |
| Bu | Butane |
| Et | Ethen |
| Pr | Propen |
| 2-Pe | 2-Penten |
| 3-He | 3-Hexen |
| H | Hochsieder |
| II | Raffinat II |
| C4 | C4-Olefine |
| C5⁺ | Olefine mit 5 oder mehr Kohlenstoffatomen |
| R1 | Reaktor |
| R2 | Reaktor |
| D1 | Destillationskolonne (wenn unter D1 ein senkrechter Strich vorgesehen ist, handelt es sich um eine Trennwandkolonne) |
| D2 | Kolonne (wenn unter D2 ein senkrechter Strich vorgesehen ist, handelt es sich um eine Trennwandkolonne) |
| D3 | Destillationskolonne |

Nachstehend wird eine Ausführungsform des erfindungsgemäßen Verfahrens beschrieben, umfassend
a) Umsetzung von 1-Buten und 2-Buten zu Propen und 2-Penten in Gegenwart eines Metathesekatalysators, der mindestens eine Verbindung eines Metalls VIb, VIIb oder VIII Nebengruppe des Periodensystems der Elemente enthält,
b) anschließendes Trennen des gebildeten Propens und 2-Pentens,
c) anschließende Umsetzung des 2-Pentens mit Ethen zu Propen und 1-Buten in Gegenwart eines Metathesekatalysators, der mindestens eine Verbindung eines Metalls der VIb, VIIb oder VIII Nebengruppe des Periodensystems der Elemente enthält,
d) anschließendes Trennen des gebildeten Propens und 1-Butens,
e) anschließendes Rückführen des gebildeten 1-Butens in Schritt a).

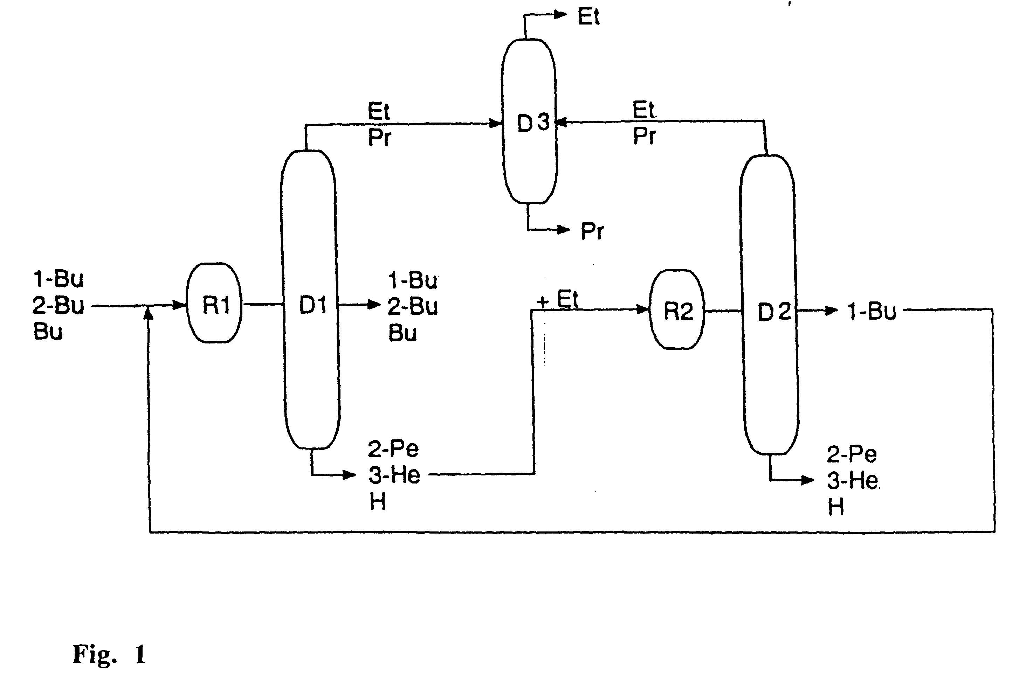

Diese Ausführungsform ist in Fig. 1 dargestellt.

In einem ersten Reaktor R1 werden in Gegenwart des erfindungsgemäßen Metathesekatalysators 1-Buten und 2-Buten zu Propen und 2-Penten umgesetzt. Dazu wird ein Raffinat II-Strom in den Reaktor geführt. An den Reaktor schließt sich eine Destillationskolonne D1 an, an deren Kopf Propen und als Nebenprodukt gebildetes Ethen entfernt werden. Nicht umgesetztes Raffinat II wird im Mittelabzug entfernt. Es kann auch teilweise in den Reaktor R1 zurückgeführt werden (in Fig. 1 nicht dargestellt). Aus dem Sumpf von D1 werden 2-Penten und als Nebenprodukt gebildetes 3-Hexen sowie Hochsieder entnommen. Der Sumpf wird sodann zusammen mit eingespeistem Ethen einem Reaktor R2 zugeführt, der wiederum einen erfindungsgemäßen Metathesekatalysator enthält. In diesem Reaktor R2 findet die Umsetzung von 2-Penten mit Ethen zu 1-Buten und Propen statt. Der Austrag aus Reaktor R2 wird einer Destillationskolonne D2 zugeführt, an deren Kopf Propen und nicht umgesetztes Ethen abgeführt werden. Im Mittelabzug wird gebildetes 1-Buten ausgetragen, und vorzugsweise zumindest teilweise zum Reaktor R1 zurückgeführt. Im Sumpf von D2 fallen nicht umgesetztes 2-Penten, sowie als Nebenprodukte 3-Hexen und Hochsieder an. Diese werden vorzugsweise in den Reaktor R2 zurückgeführt. Die aus D1 und D2 am Kopf abgeführten Gemische aus Propen und Nebenprodukt Ethen werden in einer weiteren Destillationskolonne D3 aufgetrennt. Am Kopf von D3 wird Ethen gewonnen, das vorzugsweise in den Reaktor R2 (zurück)geführt wird (nicht in Fig. 1 dargestellt), oder als Co-Crackfeed ausgeschleust wird. Das im Sumpf von D3 erhaltene Propen ist das gewünschte Umsetzungsprodukt des erfindungsgemäßen Verfahrens. D1 und D2 sind so ausgelegt, daß am Kopf der Kolonne eine leichtsiedende Phase, insbesondere eine C_{2/3}-Phase, die Ethen und Propen enthält, abgeführt wird. Als Mittelsiederphase werden C₄-Ströme abgeführt, insbesondere Butene und Butane. In der Sumpfphase werden C_{≥5}-Kohlenwasserstoffe ausgetragen.

Die Reaktoren R1 und R2 können beliebige geeignete Reaktoren sein. Sie können zur kontinuierlichen oder diskontinuierlichen Reaktionsführung dienen.

Somit können Sie gemäß einer Ausführungsform Druckgefäße, wie Druckglasgefäße, gemäß einer weiteren Ausführungsform Rohrreaktoren sein.

Gemäß einer Ausführungsform der Erfindung beträgt der Gesamtumsatz in R1 20 bis 90, vorzugsweise 50 bis 80%.

Gemäß einer Ausführungsform der Erfindung beträgt der Gesamtumsatz in R2 20 bis 100, vorzugsweise 60 bis 90%.

Vorzugsweise findet die Umsetzung in R1 in flüssiger Phase statt. Dabei werden Druck und Temperatur so gewählt, daß die Reaktionspartner in der flüssigen Phase verbleiben.

Gemäß einer Ausführungsform der Erfindung beträgt die Temperatur in R1 0 bis 150°C, vorzugsweise 20 bis 80°C. Der Druck beträgt gemäß einer Ausführungsform der Erfindung 2 bis 200 bar, vorzugsweise 5 bis 20 bar. Die Umsetzung in R2 (Ethenolyse) wird gemäß einer Ausführungsform der Erfindung bei Temperaturen von 0 bis 150°C, vorzugsweise 20 bis 80°C unter einem Ethendruck von 5 bis 200 bar, vorzugsweise 30 bis 80 bar durchgeführt. Ethen kann dabei kontinuierlich nachgepreßt werden, so daß ein konstanter Druck eingehalten wird.

Die Umsetzungen in R1 und R2 können für eine Zeit von einer Sekunde bis 10 Stunden, vorzugsweise 1 bis 60 Minuten erfolgen.

Bei den Destillationskolonnen D1 und D2 handelt es sich gemäß einer Ausführungsform der Erfindung um solche Kolonnen, die eine Auftrennung eines Kohlenwasserstoffstroms in C_{2/3}-Ströme, C₄-Ströme und C_{≥5}-Ströme erlauben. Die Kolonnen können als Trennwandkolonnen ausgeführt sein. D3 ist gemäß einer Ausführungsform der Erfindung eine Kolonne, die die Trennung von Ethen und Propen erlaubt. Gemäß einer Ausführungsform der Erfindung ist der Reaktor R1 mit der Destillationskolonne D1 kombiniert zu einer Reaktivdestillationseinrichtung. Dabei erfolgt die Umsetzung direkt in der Destillationskolonne. Der Katalysator liegt in der Reaktionskolonne vor, so daß gleichzeitig mit der Umsetzung oder unmittelbar darauffolgend die Destillation ausgeführt wird. Ein entsprechendes Verfahren ist unter der Bezeichnung "Reaktivdestillation" bekannt.

Gemäß einer Ausführungsform sind Reaktor R2 und Destillationskolonne D2 zusammengefaßt zu einer Reaktivdestillationsvorrichtung, bei der Umsetzung und Destillation kombiniert sind entsprechend der vorstehend beschriebenen Reaktivdestillation.

Gemäß einer Ausführungsform der Erfindung finden beide Umsetzungsreaktionen in Reaktivdestillationsvorrichtungen statt. Bei beiden Umsetzungen handelt es sich um Gleichgewichtsreaktionen, so daß gemäß einer Ausführungsform der Erfindung die Verfahrensprodukte zur Erzielung eines möglichst hohen Umsatzes möglichst schnell aus dem Gleichgewicht entfernt werden. Dies ist insbesondere bei Verwendung von Reaktivdestillationsvorrichtungen möglich.

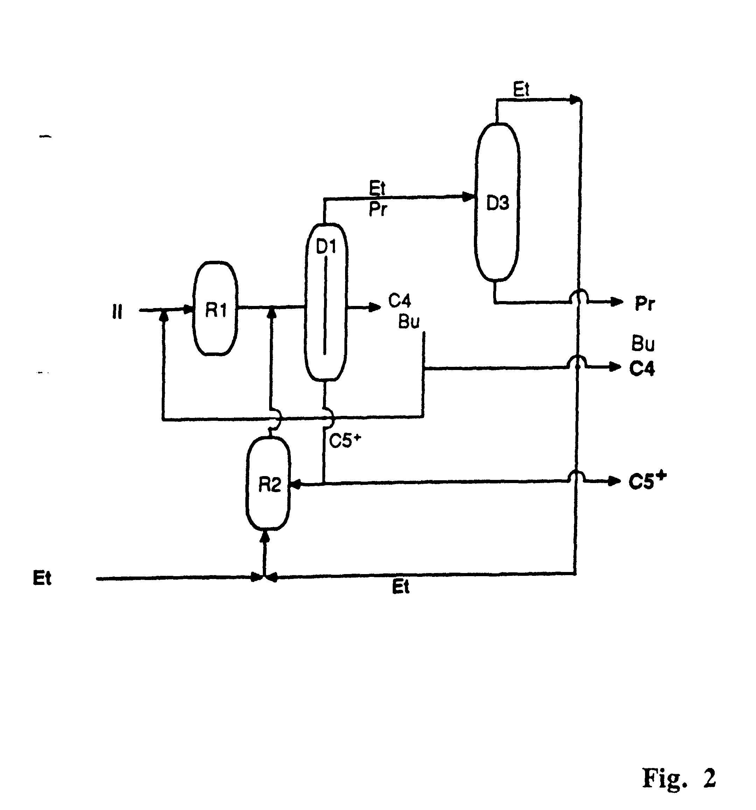

Anstelle einer normalen Destillationskolonne D1 kann eine Trennwandkolonne vorgesehen werden. Ein derartiges Verfahren ist in Figur 2 dargestellt. Das dargestellte Verfahren ist zudem gegenüber dem in Figur 1 gezeigten Verfahren modifiziert.

Wie in der vorstehend beschriebenen Ausführungsform wird im Reaktor R1 eine Metathese an einem heterogenen Metathesekatalysator durchgeführt, wobei Raffinat II eingesetzt wird. Die Destillationskolonne D1 dient der Auftrennung der bei der Metathese entstandenen Reaktionsprodukte. Die Destillationskolonne D3 dient der Trennung von Ethen und Propen. Der Reaktor R2 dient der Umsetzung von C₅⁺ -Hochsiedern mit Ethen.

Im Unterschied zur vorstehenden Ausführungsform ist die Destillationskolonne D1 als Trennwandkolonne ausgeführt. Zudem wird der Mittelsiederaustrag aus D1, der C₄-Olefine und Butane enthält, teilweise in den Feedstrom von Raffinat II zurückgeführt. Da die Destillationskolonnen D1 und D2 die gleiche Trennaufgabe bewältigen müssen, wurde in dieser Ausführungsform nur eine derartige Destillationskolonne D1 vorgesehen. Damit läßt sich der apparative Aufwand verringern. Entsprechend wurde das Reaktionsschema angepaßt: Der Hochsiederaustrag aus D1 wird in den Reaktor R2 geführt oder teilweise ausgeschleust. Der Austrag aus dem Reaktor R2 wird in die Destillationskolonne D1 geführt. Die Beschickung des Reaktors R2 mit Ethen erfolgt zum einen aus dem Leichtsiederaustrag der Destillationskolonne D3, zum anderen durch zusätzlich eingeführtes Ethen. Aus dem Verfahren werden Propen als Hauptprodukt und zudem C₄-Olefine und C₅⁺-Olefine ausgeschleust.

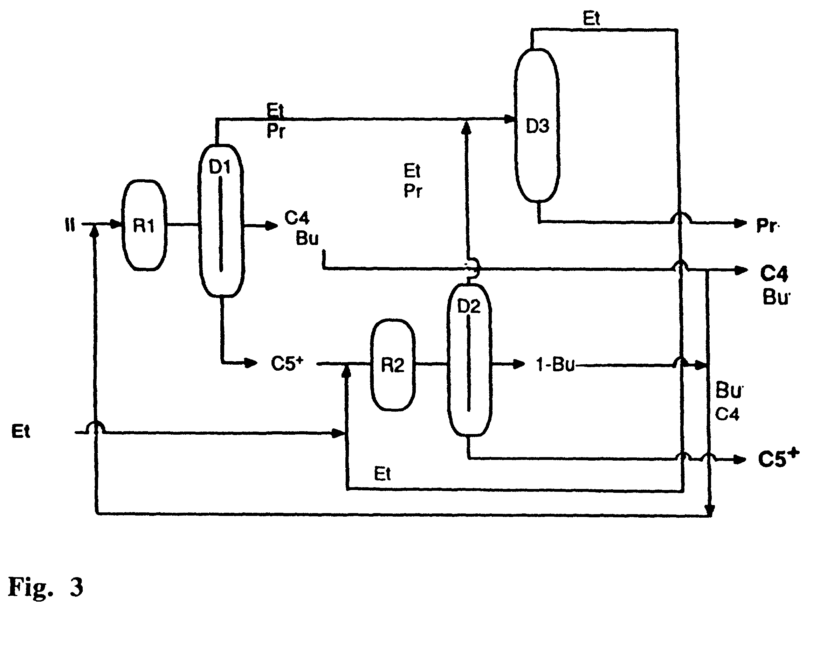

In Figur 3 ist eine Ausführungsform des erfindungsgemäßen Verfahrens dargestellt, die der in Figur 1 dargestellten Ausführungsform weitgehend entspricht. Die Kolonne D2 ist ebenso wie die Kolonne D1 als Trennwandkolonne ausgelegt.

Im Unterschied zu dem Verfahren gemäß Figur 1 wird der Mittelsiederaustrag aus D1, der C₄-Olefine und Butane enthält, teilweise ausgeschleust oder mit dem Mittelsiederaustrag aus der Kolonne D2 vereint und in den Reaktor R1 zurückgeführt. Das aus der Destillationskolonne D3 gewonnene Ethen wird in den Reaktor R2 geführt, indem die Umsetzung mit der C₅⁺-Fraktion erfolgt. Es werden wiederum Propen als Hauptprodukt und Teile der C₄-Olefinfraktion und Butane sowie die C₅⁺-Fraktion aus der Destillationskolonne D2 (auch als Co-Crackfeed) ausgeschleust.

### Katalysator

In den erfindungsgemäßen Verfahren können alle geeigneten Metathesekatalysatoren eingesetzt werden.

Gemäß einer Ausführungsform der Erfindung ist der Katalysator ein heterogener Katalysator, insbesondere ein Trägerkatalysator. Gemäß einer Ausführungsform der Erfindung enthält der Katalysator mindestens eine Verbindung eines Metalls der VIb, VIIb oder VIII Nebengruppe des Periodensystems der Elemente. Vorzugsweise enthält der Katalysator eine Rutheniumverbindung und/oder Rheniumverbindung.

Gemäß einer Ausführungsform der Erfindung handelt es sich bei der Metallverbindung um ein Metalloxid, Teiloxid mit zusätzlich vorliegenden organischen Resten oder um eine Carbonylverbindung.

Gemäß einer Ausführungsform der Erfindung wird ein homogener Katalysator eingesetzt. Der Katalysator ist dabei mindestens eine Verbindung eines Metalls der VIb, VIIb oder VIII Nebengruppe des Periodensystems der Elemente. Vorzugsweise wird Rhenium oder Ruthenium eingesetzt in den Metallverbindungen.

Gemäß einer Ausführungsform der Erfindung werden Rutheniumverbindungen eingesetzt, wie sie in WO 93/20111 und WO 96/04289 beschrieben sind.

Gemäß einer bevorzugten Ausführungsform der Erfindung wird

RuX₂(CHR)(PR'₃)₂ eingesetzt, wobei die Reste R und R' C₁₋₁₂-Alkylreste, vorzugsweise C₁₋₆-Alkylreste oder C₆₋₁₂-Arylreste sind, R¹ besonders bevorzugt ein C₃₋₈-Cycloalkylrest, insbesondere C₅- oder C₆-Cycloalkylrest ist und X ein Halogenid ist, wie Chlorid, Bromid oder Iodid.

Insbesondere wird erfindungsgemäß RuCl₂(=CHPh)(PCy₃)₂ eingesetzt, gemäß einer Ausführungsform der Erfindung als Lösung, beispielsweise in Methylenchlorid.

Vorzugsweise ist die Metallverbindung ein Oxid von Rhenium, insbesondere Re₂O₇.

### Träger

Die erfindungsgemäßen Katalysatoren enthalten gemäß einer Ausführungsform der Erfindung einen Träger. Als Träger kommen dabei insbesondere anorganische Träger zur Anwendung, wie Al₂O₃, insbesondere γ-Al₂O₃, SiO₂, Fe₂O₃, oder deren Gemische, wie SiO₂/Al₂O₃, B₂O₃/SiO₂/Al₂O₃ oder Fe₂O₃/Al₂O₃.

Der Metalloxidgehalt auf dem Träger beträgt dabei gemäß einer Ausführungsform der Erfindung 1 bis 20 Gew.-%, vorzugsweise 3 bis 15 Gew.-%, insbesondere 8 bis 12 Gew.-%, bezogen auf das Gesamtgewicht des Trägerkatalysators.

Bevorzugt wird als Katalysator Re₂O₇ auf Al₂O₃, SiO₂/Al₂O₃, SiO₂/Al₂O₃/Fe₂O₃ oder B₂O₃/Al₂O₃ verwendet. Dabei beträgt der Anteil an Metalloxid vorzugsweise 1 bis 20 Gew.-%, besonders bevorzugt 3 bis 10 Gew.-%. Gemäß einer Ausführungsform der Erfindung wird MeReO₃ anstelle von Re₂O₇ oder im Gemisch damit verwendet.

Besonders bevorzugt wird erfindungsgemäß Re₂O₇ auf Al₂O₃ verwendet.

Die Katalysatoren werden gemäß einer Ausführungsform der Erfindung frisch calciniert eingesetzt, wobei sie keiner weiteren Aktivierung, beispielsweise durch Alkylierungsmittel bedürfen. Desaktivierte Katalysatoren können erfindungsgemäß durch Abbrennen von Coke-Rückständen beispielsweise bei 550°C im Luftstrom und Abkühlung unter Argon regeneriert werden.

Die erfindungsgemäßen Umsetzungen können dabei in Gegenwart eines Lösungsmittels, beispielsweise eines Kohlenwasserstofflösungsmittels durchgeführt werden. Gemäß einer bevorzugten Ausführungsform der Erfindung werden die Umsetzungen ohne weiteres zugesetztes Lösungsmittel durchgeführt.

Die Erfindung betrifft auch Vorrichtungen zur Durchführung der beschriebenen Verfahren. Eine Vorrichtung zur Durchführung des Verfahrens gemäß Figur 2 umfaßt einen Methathesereaktor (R1) zur Umsetzung von 1-Buten mit 2-Buten, an dessen Ausgang sich eine Destillationskolonne (D1) zur Trennung von C_{2/3}-Leichtsieder-, C₄-Mittelsieder- und C₅⁺-Hochsiederphasen anschließt, die als Trennwandkolonne ausgelegt sein kann, wobei der Leichtsiederausgang in eine Kolonne (D3) zur Trennung von Ethen und Propen führt, der Mittelsiederausgang zum Reaktor (R1) oder zur Ausschleusung führt und der Hochsiederausgang in einen Reaktor (R2) zur Umsetzung von 2-Penten mit Ethen, dessen Ausgang in die Kolonne (D1) führt, oder zur Ausschleusung führt, wobei der Ethenausgang aus der Kolonne (D3) und eine Ethenzuführung in den Reaktor (R2) führen.

Eine Vorrichtung zur Durchführung des Verfahrens gemäß Figur 3 umfaßt einen Methathesereaktor (R1) zur Umsetzung von 1-Buten mit 2-Buten, an dessen Ausgang sich eine Destillationskolonne (D1) zur Trennung von C_{2/3}-Leichtsieder-, C₄-Mittelsieder- und C₅⁺-Hochsiederphasen ausschließt, die als Trennwandkolonne ausgelegt sein kann, wobei der Leichtsiederausgang in eine Kolonne (D3) zur Trennung von Ethen und Propen führt, der Mittelsiederausgang zum Reaktor (R1) oder zur Ausschleusung führt, und der Hochsiederausgang in einen Reaktor (R2) zur Umsetzung von 2-Penten mit Ethen führt, an dessen Ausgang sich eine Destillationskolonne (D2) zur Trennung von C_{2/3}-Leichtsieder-, C₄-Mittelsieder- und C₅⁺-Hochsiederphasen anschließt, die als Trennwandkolonne ausgelegt sein kann, wobei der Leichtsiederausgang in die Kolonne (D3) führt, der Mittelsiederausgang gemeinsam mit dem Mittelsiederausgang von (D1) zum Reaktor (R1) führt und der Hochsiederausgang zur Ausschleusung führt, wobei der Ethenausgang aus der Kolonne (D3) und eine Ethenzuführung in den Reaktor (R2) führen.

Nachstehend wird die Erfindung anhand von Beispielen näher erläutert.

### Beispiele 1 bis 6

### Diskontinuierliche Synthese von Propen aus Raffinat II

In einem 100 ml fassenden Druckbehälter wurden 50 ml Raffinat II (mit einem Verhältnis von 1-Buten zu 2-Buten von 1:2) mit 10 g eines frisch calcinierten Heterogenkatalysators bei 60 °C und 7 bar gerührt. Die eingesetzten Katalysatoren sind in der nachstehenden Tabelle 1 aufgeführt. In Zeitintervallen von 5 Minuten wurden Proben entnommen und gaschromatographisch untersucht. Nachstehend sind die in der Gemischzusammensetzung nach 15 Minuten vorliegenden und darauf bezogenen 1-Butenumsätze und Propen-Selektivitäten aufgeführt.

**Tabelle**

| BEISPIEL | KATALYSATOR | UMSATZ 1-BUTEN | SELEKTIVITÄT PROPEN |
|---|---|---|---|
| 1 | 3% Re₂O₇/Al₂O₃ | 63% | 93% |
| 2 | 10% Re₂O₇/Al₂O₃ | 85% | 92% |
| 3 | 3% Re₂O₇/SiO₂/Al₂O₃ | 70% | 91% |
| 4 | 3% Re₂O₇/SiO₂/Al₂O₃/Fe₂O₃ | 38% | 88% |
| 5 | 3% Re₂O₇/B₂O₃/Al₂O₃ | 68% | 92% |
| 6 | 3% MeReO₃/Al₂O₃ | 41% | 89% |

Für alle Katalysatoren ergeben sich Propen-Selektivitäten in bezug auf 1-Buten von mindestens 88%. Insbesondere der Katalysator aus 3% Re₂O₇ auf Al₂O₃ zeigte eine Selektivität von 93%. Der Umsatz an 1-Buten lag im Bereich von 38 bis 85%.

### Beispiel 7

### Homogenkatalysierte Synthese von Propen aus Raffinat II

In einem 100 ml fassenden Druckbehälter wurden 50 ml Raffinat II (mit einem 1-Buten-zu-2-Buten-Verhältnis von 1:2) bei Raumtemperatur mit einer Lösung von 31 mg (0,04 mmol) RuCl₂(=CHPh)(PCy₃)₂ in 5 ml Methylenchlorid versetzt. In Zeitintervallen von 2 min wurden Proben entnommen und gaschromatographisch untersucht. Der 1-Buten-Umsatz nach 10 Minuten betrug 81%, die Propen-Selektivität 90%.

### Beispiel 8

Die gemäß Beispiel 1 bis 7 anfallenden Reaktionsgemische wurden destillativ aufgetrennt, wobei als Hochsiederaustrag im Sumpf 2-Penten mit 3-Hexen als Nebenprodukt erhalten wurde. In einem 100 ml fassenden Druckbehälter wurden 50 ml dieses Hochsiederaustrages in Gegenwart von 10 g des 3% Re₂O₇/Al₂O₃-Katalysators aus Beispiel 2 mit 50 bar Ethen behandelt. Dabei wurde der Druck durch kontinuierliches Nachpressen von Ethen bei einer Temperatur von 60°C aufrechterhalten. In Zeitintervallen von 2 Minuten wurden Proben entnommen und gaschromatographisch untersucht. Nach 20 Minuten hatte sich eine Gemisch-Zusammensetzung eingestellt mit
Umsatz (2-Penten und 3-Hexen) = 64%
Selektivität (1-Buten und Propen) = 96%.

Aus den vorstehenden Ergebnissen geht hervor, daß das erfindungsgemäße Verfahren sehr gut geeignet ist zur Herstellung von Propen aus Raffinat II-Strömen (2-Buten und 1-Buten).

### Beispiel 9

### Kontinuierliche Synthese von Propen aus Raffinat II

Raffinat II wurde bei einer Temperatur von 60 °C und einem Druck von 10 bar kontinuierlich bei unterschiedlichen Verweilzeiten über ein mit einem Re₂O₇/Al₂O₃-Heterogenkontakt bestücktes Strömungsrohr geleitet. Es wurde der Katalysator aus Beispiel 1 verwendet. Der Reaktionsaustrag wurde nach der Entspannung gaschromatographisch untersucht. Die Ergebnisse sind in der nachstehenden Tabelle dargestellt.

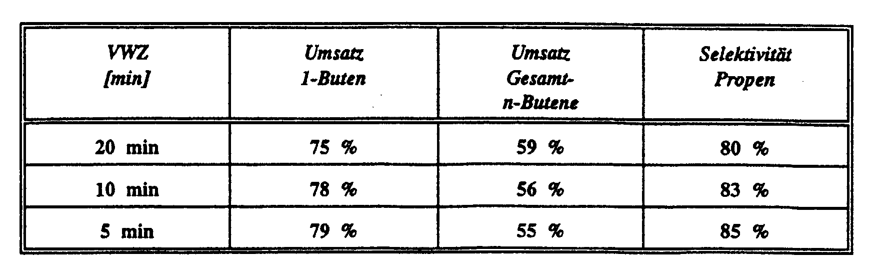

Die erzielten Raum-Zeit-Ausbeuten liegen im Bereich um 200 - 2000 g Propen / 1 h.

### Beispiel 10

Es wurden die Verfahren gemäß Beispielen 1 bis 9 wiederholt, allerdings enthielt der eingesetzte Feed etwa 40 bis 50 ppm Wasser und 60 bis 80 ppm Oxygenates. Der Feed wurde in einer Verfahrensvariante direkt umgesetzt, m einer weiteren Verfahrensvariante vor der Umsetzung gasförmig über Molsieb 13 x (Nax-Zeolith) geführt, wobei Wasser und Oxygenates entfernt wurden. Die Standzeit des Metathesekatalysators wurde beim Führen des Feed über Molsieb 13 x um den Faktor 10 bis 20 gegenüber der ersten Verfahrensvariante verlängert.

## Patentansprüche

1. Verfahren zur Herstellung von Propen durch
a) Umsetzung von 1-Buten und 2-Buten zu Propen und 2-Penten in Gegenwart eines Metathesekatalysators, der mindestens eine Verbindung eines Metalls VIb, VIIb oder VIII Nebengruppe des Periodensystems der Elemente enthält,
b) anschließendes Trennen des gebildeten Propens und 2-Pentens,
c) anschließende Umsetzung des 2-Pentens mit Ethen zu Propen und 1-Buten in Gegenwart eines Metathesekatalysators, der mindestens eine Verbindung eines Metalls der VIb, VIIb oder VIII Nebengruppe des Periodensystems der Elemente enthält,
d) anschließendes Trennen des gebildeten Propens und 1-Butens,
e) anschließendes Rückführen des gebildeten 1-Butens in Schritt a).

2. Verfahren nach Anspruch 1, wobei Schritt b) eine Destillation ist, die in einer Trennwandkolonne durchgeführt werden kann, in der eine Propen enthaltende Leichtsiederphase, gegebenenfalls eine Butene enthaltende Mittelsiederphase und eine 2-Penten enthaltende Sumpfphase erhalten werden,
und/oder
wobei Schritt d) eine Destillation ist, die in einer Trennwandkolonne durchgeführt werden kann, in der eine Propen enthaltende Leichtsiederphase, eine 1-Buten enthaltende Mittelsiederphase und gegebenenfalls eine 2-Penten enthaltende Sumpfphase erhalten werden, wobei Schritte b) und d) in einer Destillationskolonne durchgeführt werden können.

3. Verfahren nach Anspruch 1 oder 2, wobei die Umsetzung in Schritten a) und/oder c) nicht vollständig erfolgt und in Schritt b) und/oder d) eine C_{2/3} enthaltende Leichtsiederphase, eine C₄-Mittelsiederphase und eine C_{≥5} enthaltende Sumpfphase erhalten werden,
wobei die gegebenenfalls zusammengefaßten Leichtsiederphasen durch Destillation in C₂- und C₃-Phasen getrennt werden und die C₂-Phase in Schritt c) zurückgeführt wird,
die gegebenenfalls zusammengefaßten Mittelsiederphasen zumindest teilweise in Schritt a) zurückgeführt werden und die gegebenenfalls zusammengefaßten Sumpfphasen zumindest teilweise in Schritt c) zurückgeführt werden.

4. Verfahren nach einem der Ansprüche 1 bis 3, wobei 1-Buten und 2-Buten als Gemisch eines C₄-Stroms, vorzugsweise aus einem Cracker oder einer Raffinerie, insbesondere als Raffinat II eingesetzt werden, wobei der C₄-Strom vor der Umsetzung zur Reinigung und Entfernung von Spuren an Wasser, Sauerstoff, Schwefel, Stickstoff, Phosphor oder Halogenen über Absorbermaterialien geleitet werden kann.

5. Verfahren nach Anspruch 4, wobei das Verhältnis von Molanteil 1-Buten zu Molanteil 2-Buten, 10:1 bis 1:10, beträgt.

6. Verfahren nach einem der Ansprüche 1 bis 5, wobei ein homogener oder heterogener Metathesakatalysator eingesetzt wird, der eine Rhenium- oder Rutheniumverbindung enthält, vorzugsweise ein Rheniumoxid auf einem anorganischen Träger.

7. Verfahren nach Anspruch 6, wobei der Metathesekatalysator Re₂O₇ auf einem Al₂O₃-Träger enthält oder daraus besteht, wobei der Rheniumoxidgehalt 1 bis 20 Gew.-%, vorzugsweise 3 bis 10 Gew.-%, bezogen auf das Gesamtgewicht des Katalysators, beträgt.

8. Verfahren nach einem der Ansprüche 1 bis 7, wobei die Umsetzung von 1-Buten und 2-Buten und/oder die Umsetzung von 2-Penten mit Ethen als Reaktivdestillation durchgeführt werden.

9. Vorrichtung zur Durchführung eines Verfahrens nach einem der Ansprüche 1 bis 8, umfassend einen Methathesereaktor (R1) zur Umsetzung von 1-Buten mit 2 Buten, an dessen Ausgang sich eine Destillationskolonne (D1) zur Trennung von C_{2/3}-Leichtsieder-, C₄-Mittelsieder- und C₅⁺-Hochsiederphasen anschließt, die als Trennwandkolonne ausgelegt sein kann, wobei der Leichtsiederausgang in eine Kolonne (D3) zur Trennung von Ethen und Propen führt, der Mittelsiederausgang zum Reaktor (R1) oder zur Ausschleusung führt und der Hochsiederausgang in einen Reaktor (R2) zur Umsetzung von 2-Penten mit Ethen, dessen Ausgang in die Kolonne (D1) führt, oder zur Ausschleusung führt, wobei der Ethenausgang aus der Kolonne (D3) und eine Ethenzuführung in den Reaktor (R2) führen.

10. Vorrichtung zur Durchführung eines Verfahrens nach einem der Ansprüche 1 bis 8, umfassend einen Methathesereaktor (R1) zur Umsetzung von 1-Buten mit 2-Buten, an dessen Ausgang sich eine Destillationskolonne (D1) zur Trennung von C_{2/3}-Leichtsieder-, C₄-Mittelsieder- und C₅⁺-Hochsiederphasen ausschließt, die als Trennwandkolonne ausgelegt sein kann, wobei der Leichtsiederausgang in eine Kolonne (D3) zur Trennung von Ethen und Propen führt, der Mittelsiederausgang zum Reaktor (R1) oder zur Ausschleusung führt, und der Hochsiederausgang in einen Reaktor (R2) zur Umsetzung von 2-Penten mit Ethen führt, an dessen Ausgang sich eine Destillationskolonne (D2) zur Trennung von C_{2/3}-Leichtsieder-, C₄-Mittelsieder- und C₅⁺-Hochsiederphasen anschließt, die als Trennwandkolonne ausgelegt sein kann, wobei der Leichtsiederausgang in die Kolonne (D3) führt, der Mittelsiederausgang gemeinsam mit dem Mittelsiederausgang von (D1) zum Reaktor (R1) führt und der Hochsiederausgang zur Ausschleusung führt, wobei der Ethenausgang aus der Kolonne (D3) und eine Ethenzuführung in den Reaktor (R2) führen.

## Claims

1. A process for preparing propene by
a) reacting 1-butene and 2-butene to give propene and 2-pentene in the presence of a metathesis catalyst comprising at least one compound of a metal of transition group VIb, VIIb or VIII of the Periodic Table of the Elements,
b) subsequently separating the propene and 2-pentene formed,
c) subsequently reacting the 2-pentene with ethene to give propene and 1-butene in the presence of a metathesis catalyst comprising at least one compound of a metal of transition group VIb, VIIb or VIII of the Periodic Table of the Elements,
d) subsequently separating the propene and 1-butene formed,
e) subsequently returning the 1-butene formed to step a).

2. A process as claimed in claim 1, wherein step b) is a distillation which can be carried out in a dividing wall column in which a propene-containing low-boiling phase, possibly a butene-containing intermediate-boiling phase and a 2-pentene-containing bottom phase are obtained,
and/or
wherein step d) is a distillation which can be carried out in a dividing wall column in which a propene-containing low-boiling phase, a 1-butene-containing intermediate-boiling phase and possibly a 2-pentene-containing bottom phase are obtained, wherein steps b) and d) can be carried out in a distillation column.

3. A process as claimed in claim 1 or 2, wherein the reaction in steps a) and/or c) is not carried to completion and in step b) and/or d) a C_{2/3}-containing low-boiling phase, a C₄ intermediate-boiling phase and a C_{≥5}-containing bottom phase are obtained,
wherein the low-boiling phases, which may be combined, are separated by distillation into C₂ and C₃ phases and the C₂ phase is returned to step c),
at least some of the intermediate-boiling phases, which may be combined, are returned to step a) and at least some of the bottom phases, which may be combined, are returned to step c).

4. A process as claimed in any of claims 1 to 3, wherein 1-butene and 2-butene are used as a mixture in a C₄ stream, preferably from a cracker or a refinery, in particular as raffinate II, wherein the C₄ stream can be passed over absorber materials before the reaction to purify it and to remove traces of water, oxygen, sulfur, nitrogen, phosphorus or halogens.

5. A process as claimed in claim 4, wherein the molar ratio of 1-butene to 2-butene is from 10:1 to 1:10.

6. A process as claimed in any of claims 1 to 5, wherein use is made of a homogeneous or heterogeneous metathesis catalyst comprising a rhenium or ruthenium compound, preferably a rhenium oxide, on an inorganic support.

7. A process as claimed in claim 6, wherein the metathesis catalyst comprises or consists of Re₂O₇ on an Al₂O₃ support, with the rhenium oxide content being from 1 to 20 % by weight, preferably from 3 to 10 % by weight, based on the total weight of the catalyst.

8. A process as claimed in any of claims 1 to 7, wherein the reaction of 1-butene and 2-butene and/or the reaction of 2-pentene with ethene are carried out as a reactive distillation.

9. An apparatus for carrying out a process as claimed in any of claims 1 to 8, comprising a metathesis reactor (R1) for reacting 1-butene with 2-butene whose outlet leads to a distillation column (D1), which can be configured as a dividing wall column, for separating C_{2/3} low-boiling, C₄ intermediate-boiling and C₅⁺ high-boiling phases, where the low boiler outlet leads to a column (D3) for separating ethene and propene, the intermediate boiler outlet leads to the reactor (R1) or to discharge and the high boiler outlet leads to a reactor (R2) for reacting 2-pentene with ethene whose outlet leads to the column (D1) or to discharge, where the ethene outlet from the column (D3) and an ethene feed line lead to the reactor (R2).

10. An apparatus for carrying out a process as claimed in any of claims 1 to 8, comprising a metathesis reactor (R1) for reacting 1-butene with 2-butene whose outlet leads to a distillation column (D1), which can be configured as a dividing wall column, for separating C_{2/3} low-boiling, C₄ intermediate-boiling and C₅⁺ high-boiling phases, where the low boiler outlet leads to a column (D3) for separating ethene and propene, the intermediate boiler outlet leads to the reactor (R1) or to discharge and the high boiler outlet leads to a reactor (R2) for reacting 2-pentene with ethene whose outlet leads to a distillation column (D2), which can be configured as a dividing wall column, for separating C_{2/3} low-boiling, C₄ intermediate-boiling and C₅⁺ high-boiling phases, where the low boiler outlet leads to the column (D3), the intermediate boiler outlet together with the intermediate boiler outlet from (D1) leads to the reactor (R1) and the high boiler outlet leads to discharge, where the ethene outlet from the column (D3) and an ethene feed line lead to the reactor (R2).

## Revendications

1. Procédé pour la préparation du propène par
a) Conversion de 1-butène et de 2-butène en propène et 2-pentène en présence d'un catalyseur métathétique, qui contient au moins un composé d'un métal des sous-groupes Vlb, Vllb ou VIII du système périodique des éléments,
b) Séparation subséquente du propène et du 2-pentène formés,
c) Réaction subséquente du 2-pentène avec de l'éthène pour former du propène et du 1-butène, en présence d'un catalyseur métathétique, contenant au moins un composé d'un métal des sous-groupes Vlb, Vllb ou VIII du système périodique des éléments,
d) Séparation subséquente du propène et du 1-butène formés,
e) Recyclage subséquent du 1-butène formé dans l'étape a).

2. Procédé selon la revendication 1, caractérisé en ce que l'étape b) est une distillation, qui peut être entreprise dans une colonne à séparation dans laquelle on obtient une fraction de bas point d'ébullition contenant du propène, éventuellement une fraction de point d'ébullition moyen contenant des butènes et une fraction de fond de colonne contenant du 2-pentène,
et/ou
en ce que l'étape d) est une distillation, qui peut être entreprise dans une colonne à séparation dans laquelle on obtient une fraction de bas point d'ébullition contenant du propène, une fraction de point d'ébullition moyen contenant du 1-butène et éventuellement une fraction de fond de colonne contenant du 2-pentène, les étapes b) et d) pouvant être entreprises dans une colonne de distillation.

3. Procédé selon la revendication 1 ou 2, caractérisé en ce que la réaction aux étapes a) et/ou c) n'est pas complète et en ce que l'on obtient aux étapes b) et/ou d) une fraction de bas point d'ébullition contenant du C_{2/3}, une fraction de point d'ébullition moyen en C₄ et une fraction de fond de colonne en C_{≥5},
et caractérisé en ce que les phases de bas point d'ébullition éventuellement rassemblées sont séparées par distillation en phases C₂ et C₃ et que la phase C₂ est recyclée dans l'étape c),
et que les phases de point d'ébullition moyen éventuellement rassemblées sont au moins partiellement recyclées dans l'étape a) et que les phases de fond de colonne éventuellement rassemblées sont au moins partiellement recyclées dans l'étape c).

4. Procédé selon l'une des revendications 1 à 3, caractérisé en ce que l'on met en oeuvre le 1-butène et le 2-butène sous la forme d'un mélange de courants de C₄, provenant de préférence d'une installation de craquage ou de raffinage, en particulier sous forme de raffinat Il, caractérisé en ce que l'on peut faire passer le courant de C₄, avant la réaction, sur des matériaux absorbants aux fins d'épuration et d'élimination de traces d'eau, d'oxygène, de soufre, d'azote, de phosphore ou d'halogènes.

5. Procédé selon la revendication 4, caractérisé en ce que le rapport de la fraction molaire du 1-butène à la fraction molaire du 2-butène est de 10:1 à 1:10.

6. Procédé selon l'une des revendications 1 à 5, caractérisé en ce que l'on met en oeuvre un catalyseur métathétique homogène ou hétérogène contenant un composé de rhénium ou de ruthénium, de préférence un oxyde de rhénium sur un support inorganique.

7. Procédé selon la revendication 6, caractérisé en ce que le catalyseur métathétique est ou contient du Re₂O₇ sur un support d'Al₂O₃, la fraction d'oxyde de rhénium étant de 1 à 20% en poids, de préférence de 3 à 10% en poids, par rapport au poids total du catalyseur.

8. Procédé selon l'une des revendications 1 à 7, caractérisé en ce que la réaction du 1-butène et du 2-butène et/ou la réaction du 2-pentène avec l'éthène est entreprise sous la forme d'une distillation réactive.

9. Equipement pour la réalisation d'un procédé selon l'une des revendications 1 à 8, comprenant un réacteur de métathèse (R1) pour la réaction du 1-butène avec le 2-butène, à la sortie duquel est reliée une colonne de distillation (D1) pour la séparation de phases de bas point d'ébullition en C_{2/3}, de point d'ébullition moyen en C₄ et de haut point d'ébullition en C₅⁺, qui peut être équipée en colonne à séparation, la sortie à bas point d'ébullition menant à une colonne (D3) pour la séparation de l'éthène et du propène, la sortie à point d'ébullition moyen menant à un réacteur (R1) ou à un éclusage au dehors, et la sortie à haut point d'ébullition menant à un réacteur (R2) pour la réaction du 2-pentène avec l'éthène, dont la sortie aboutit à la colonne (D1) ou à un éclusage au dehors, caractérisé en ce que la sortie d'éthène de la colonne (D3) et une alimentation d'éthène aboutissent au réacteur (R2).

10. Equipement pour la réalisation d'un procédé selon l'une des revendications 1 à 8, comprenant un réacteur de métathèse (R1) pour la réaction du 1-butène avec le 2-butène, à la sortie duquel est reliée une colonne de distillation (D1) pour la séparation de phases de bas point d'ébullition en C_{2/3}, de point d'ébullition moyen en C₄ et de haut point d'ébullition en C₅⁺, qui peut être équipée en colonne à séparation, la sortie à bas point d'ébullition menant à une colonne (D3) pour la séparation de l'éthène et du propène, la sortie à point d'ébullition moyen menant à un réacteur (R1) ou à un éclusage au dehors, et la sortie à haut point d'ébullition menant à un réacteur (R2) pour la réaction du 2-pentène avec l'éthène, dont la sortie est reliée à une colonne de distillation (D2) pour la séparation de phases de bas point d'ébullition en C_{2/3}, de point d'ébullition moyen en C₄ et de haut point d'ébullition en C₅⁺, qui peut être équipée en colonne à séparation, la sortie à bas point d'ébullition menant à une colonne (D3), la sortie à point d'ébullition moyen menant conjointement avec la sortie à point d'ébullition moyen de (D1) au réacteur (R1) et la sortie à haut point d'ébullition menant à un éclusage au dehors, caractérisé en ce que la sortie d'éthène de la colonne (D3) et une alimentation d'éthène sont envoyées au réacteur (R2).
